# EUROPÄISCHE PATENTSCHRIFT

(11) **EP 3 691 729 B1**
(45) Veröffentlichungstag und Bekanntmachung des Hinweises auf die Patenterteilung: **23.07.2025**
(21) Anmeldenummer: 18778866.6
(22) Anmeldetag: 20.09.2018
(51) Int. Cl.: A61M 21/00, A63F 13/213, G01C 21/36, G02B 27/01, G06V 20/56, H04N 21/81

(54) **KINETOSEFREIES BETRACHTEN EINES DIGITALEN INHALTS IN EINEM FAHRZEUG**
VIEWING DIGITAL CONTENT IN A VEHICLE WITHOUT SUFFERING FROM MOTION SICKNESS
OBSERVATION SANS CINÉTOSE D'UN CONTENU NUMÉRIQUE DANS UN VÉHICULE

(30) Priorität: 04.10.2017 DE 102017217592
(43) Veröffentlichungstag der Anmeldung: 12.08.2020
(73) Patentinhaber: Audi AG, 85045 Ingolstadt (DE)
(72) Erfinder: PROFENDINER, Daniel, 85049 Ingolstadt (DE)
(74) Vertreter: RDL Patentanwälte PartG mbB
(86) Internationale Anmeldenummer: PCT/EP2018/075497
(87) Internationale Veröffentlichungsnummer: WO 2019/068477

(56) Entgegenhaltungen:
- EP-A1- 2 933 707
- DE-A1- 102013 021 137
- DE-A1- 102014 019 579
- DE-A1- 102014 213 021
- DE-A1- 102015 004 749
- DE-A1- 102015 011 616
- DE-C1- 10 156 219
- JP-A- 2017 146 963
- US-A1- 2014 362 113
- US-A1- 2015 097 860
- US-A1- 2016 238 852
- US-A1- 2017 227 765
- US-A1- 2017 251 176
- US-A1- 2017 254 659
- EDUARDO CUERVO: "BEYOND REALITY", GETMOBILE: MOBILE COMPUTING AND COMMUNICATIONS, ACM, 2 PENN PLAZA, SUITE 701 NEW YORK NY 10121-0701 USA, vol. 21, no. 2, 4 August 2017 (2017-08-04), pages 9 - 15, XP058366764, ISSN: 2375-0529, DOI: 10.1145/3131214.3131218

## Beschreibung

Die vorliegende Erfindung betrifft ein System zum kinetosefreien Betrachten eines digitalen Inhalts in einem Fahrzeug, welches ein Fahrzeug mit einer Steuerungseinheit und mehreren mit der Steuerungseinheit verbundenen Sensoren und eine mit der Steuerungseinheit drahtlos verbindbare Datenbrille (Head Mounted Device, HMD) zum Darstellen einer den digitalen Inhalt integrierenden virtuellen 3D-Szene umfasst.

Die Insassen eines Fahrzeugs sind während einer Fahrt unterschiedlichen Beschleunigungen ausgesetzt. Zu den Beschleunigungen zählen zum einen in eine Fahrtrichtung des Fahrzeugs wirkende lineare Beschleunigungen (Längsbeschleunigungen) des Fahrzeugs, welche durch Gasgeben oder Bremsen verursacht sind. Zum anderen erfahren die Insassen rotatorische Beschleunigungen (Querbeschleunigungen), welche je nach räumlicher Orientierung einer Drehachse bezogen auf die Fahrtrichtung des Fahrzeugs als Nicken (Drehachse horizontal und senkrecht zur Fahrtrichtung), Rollen (Drehachse parallel zur Fahrtrichtung) oder Gieren (Drehachse vertikal und senkrecht zur Fahrtrichtung) bezeichnet werden. Rotatorische Beschleunigungen werden unmittelbar durch Lenken und durch die Topologie des Untergrunds und mittelbar als federnde Antwort auf sämtliche vorgenannten direkten Beschleunigungen bewirkt.

Ein Insasse eines Fahrzeugs kann während einer Fahrt in dem Fahrzeug unter Schwindel, Kopfschmerz, Herzfrequenzanstieg, Übelkeit oder Erbrechen leiden (Kinetose), wenn die ihm über die Augen (Gesichtssinn) vermittelte visuelle Wahrnehmung von Position, Orientierung und Beschleunigung nicht konsistent zu der über den Vestibularapparat (Gleichgewichtssinn) vermittelten Wahrnehmung derselben Größen ist.

Eine solche Diskrepanz kann beispielsweise bei einem Insassen in dem Fahrzeug auftreten, welcher während der Fahrt ein Buch liest oder einen digitalen Inhalt auf einem mobilen Endgerät wie einem Smartphone oder einem Tablet betrachtet. Dabei fixiert der Insasse mit gesenktem Blick eine Seite eines möglichst ruhig gehaltenen Buchs bzw. eine Anzeige eines möglichst ruhig gehaltenen mobilen Endgeräts, während sein Gleichgewichtssinn fortwährend durch fahrtbedingte Beschleunigungen des Fahrzeugs stimuliert wird. Aber auch ein Insasse, der weder ein Buch liest noch einen Anzeige eines mobilen Endgeräts betrachtet, dessen visuelle Wahrnehmung einer Umgebung des Fahrzeugs aber eingeschränkt oder behindert ist - beispielsweise ist der freie Blick nach vorn eines hinten im Fahrzeug sitzenden Insassen durch vordere Fahrzeugsitze und Kopfstützen teilweise verstellt - kann von einer Kinetose betroffen sein.

Die US 2006/0015000 A1 offenbart ein System, ein Verfahren und eine Vorrichtung für ein Fahrzeug zum Schutz von hinten sitzenden Insassen des Fahrzeugs vor Kinetose. Das System umfasst eine mit dem Fahrzeug verbundene und nach vorn gerichtete Außenkamera und eine an einer Hinterseite einer vorderen Kopfstütze des Fahrzeugs angebrachte Anzeigeeinheit, die im Blickfeld eines hinten sitzenden Insassen angeordnet ist. Die Anzeigeeinheit macht einen von der Außenkamera aufgenommenen Videostrom für den Insassen kontinuierlich sichtbar. Dabei ist die von der Außenkamera aufgenommene Umgebung vor dem Fahrzeug jederzeit unmittelbar von der räumlichen Lage und Orientierung des Fahrzeugs abhängig und spiegelt so sämtliche während der Fahrt auftretenden Beschleunigungen in Echtzeit wieder, so dass die Gefahr einer Kinetose für den hinten sitzenden Insassen verringert oder beseitigt ist. Der Videostrom kann auch als Hintergrund eines von der Anzeigeeinheit im Vordergrund dargestellten statischen Texts dienen. Dann kann der hinten sitzende Insasse gleichzeitig den Text lesen und mit den vestibulären Beschleunigungswahrnehmungen konsistente visuelle Reize erhalten.

Bei dieser Lösung ist allerdings die Blickrichtung des hinten sitzenden Insassen durch die Position der Anzeigeeinheit vorgegeben. Im Unterschied zu einem Buch, welches gewöhnlich mit geneigtem Kopf gelesen wird, erfolgt die Lektüre mit geradeaus gerichtetem Blick, wodurch der Insasse leicht durch Reize aus dem Fahrzeug oder einer Umgebung des Fahrzeugs abgelenkt werden kann. Zudem eignet sich die Lösung in erster Linie für hinten sitzende Insassen, da ein entsprechendes Anbringen der Anzeigeeinheit für einen vorn sitzenden Insassen erschwert ist. Dieser Nachteil kann aber vermieden werden.

Beispielsweise offenbaren die US 2016/238852 A1 und die US 2017/227765 A1 jeweils eine Datenbrille (Head Mounted Display, HMD), welche als Anzeigeeinheit in einem Fahrzeug geeignet ist.

So offenbart die DE 10 2014 019 579 A1 ein System zum Betreiben einer Anzeigeeinheit in einem Fahrzeug. Die Anzeigeeinheit kann als Datenbrille ausgebildet sein und einem die Datenbrille tragenden Insassen des Fahrzeugs einen Text zur Lektüre sichtbar machen. Das Fahrzeug weist eine Mehrzahl von Sensoren zum Erfassen von Bewegungsinformationen des Fahrzeugs auf. Die Anzeigeeinheit ist konfiguriert, die Bewegungsinformationen zu empfangen und einen dezenten Hintergrund für den Text darzustellen, welcher abhängig von den empfangenen Bewegungsinformationen in Echtzeit konsistent zu den Beschleunigungen des Fahrzeugs berechnet wird. Auf diese Weise ist für den Träger der Datenbrille während der Lektüre des Texts eine Gefahr einer Kinetose reduziert.

Ähnliche Systeme werden in der DE 10 2014 019579 A1, der DE 101 56 219 C1, der JP 2017 146 963 A und der EP 2 933 717 A1 beschrieben.

Die US 2014/362113 A1 offenbart eine HMD-Anzeige, mit der ein Insasse eines Fahrzeugs einen virtuellen Videobildschirm betrachten kann, der von einer Randregion mit sich bewegenden Gegenständen umgeben wird. Die Gegenstände bewegen sich in die Richtung, in die sich wirkliche Gegenstände zu bewegen scheinen würden, wenn der Benutzer keine HMD-Anzeige tragen würde. Diese Druckschrift offenbart, als Teil eines HMD: eine Videoquelle, die Videomaterial an einem Bild-Renderer zur Erzeugung eines darzustellenden Videos übergibt; einen Bewegungssensor; und einen Generator zusätzlicher Bilder wie die sich bewegenden Gegenstände. Dem Fahrzeug kann ein GPS-Sensor zugeordnet sein, der drahtlos mit dem HMD kommuniziert. Als Zweck wird die Vermeidung von Übelkeit bei Benutzung des HMD im Fahrzeug offenbart.

Die DE 101 56 219 C1 offenbart dagegen ein Verfahren zum Reduzieren einer Kinetose beim Betrachten von Bewegtbildern in einem Fahrzeug. Eine für das Verfahren geeignete Vorrichtung umfasst eine am Kopf eines Insassen angebrachte Anzeigeeinheit (Head Mounted Device, HMD) mit sechs Beschleunigungssensoren, einem Positions-/Orientierungssensor (Six Degress Of Freedom, 6DOF Tracker) sowie einer optionalen Minikamera. Die Anzeigeeinheit ist mit einem in dem Fahrzeug separat von der Anzeigeeinheit angeordneten Rechner drahtlos oder kabelgebunden gekoppelt. Der Rechner ist konfiguriert, Daten einer DVD (Digital Versatile Disc), eines Videos, einer VR-Anwendung (Virtual Reality) oder einer AR-Anwendung (Augmented Reality) abzuspielen, abhängig von Signalen der Sensoren der Anzeigeeinheit rechnerisch zu transformieren und auf die Anzeigeeinheit derart auszugeben, dass die visuelle Wahrnehmung des Trägers der Anzeigeeinheit konsistent mit seiner vestibulären Wahrnehmung ist.

Dieses Verfahren erfordert eine hohe Bandbreite für die Übertragung der Bewegtbildströme von dem Rechner zu der Anzeigeeinheit. Bei einer kabelgebundenen Kopplung der Anzeigeeinheit an den Rechner ist eine ausreichende Bandbreite unproblematisch realisierbar. Allerdings geht diese Variante wegen des Kabels mit einer erschwerten Handhabung der Anzeigeeinheit einher. Bei der drahtlosen Kopplung ist dagegen eine einfache Handhabung unproblematisch gegeben, während bei der Übertragung der Bewegtbildströme im Falle hoher Auflösung und großer Bildänderungsrate eine ausreichende Bandbreite nicht immer sichergestellt werden kann. Diese Problematik ist verstärkt, wenn gleichzeitig mehrere Insassen des Fahrzeugs jeweils eine Anzeigeeinheit nutzen und unterschiedliche Bewegtbildströme ansehen.

Der Erfindung liegt daher die Aufgabe zugrunde, ein verbessertes System zum Reduzieren einer Kinetose beim Betrachten eines digitalen Inhalts in einem Fahrzeug zur Verfügung zu stellen, welches für die Insassen einfach handhabbar ist und mit einer geringen Bandbreite auskommt. Darüber hinaus ist es Aufgabe der Erfindung, ein verbessertes Verfahren zum Darstellen eines digitalen Inhalts in einem Fahrzeug anzugeben.

Ein Gegenstand der vorliegenden Erfindung ist ein System zum kinetosefreien Betrachten eines digitalen Inhalts in einem Fahrzeug. Das System umfasst ein Fahrzeug mit einer Steuerungseinheit und mehreren mit der Steuerungseinheit verbundenen Sensoren und eine mit der Steuerungseinheit drahtlos verbindbare Datenbrille zum Darstellen einer den digitalen Inhalt integrierenden virtuellen 3D-Szene.

Erfindungsgemäß ist die Steuerungseinheit des Fahrzeugs konfiguriert, aus von den Sensoren empfangenen Sensordaten Steuergrößen zu berechnen und die Steuergrößen an die Datenbrille zu übertragen. Die Steuerungseinheit verarbeitet die Sensordaten also vor, indem sie aus diesen Steuergrößen für die Datenbrille berechnet. Die Vorverarbeitung der Sensordaten durch die Steuerungseinheit kann zu einer Verringerung des zu übertragenden Datenvolumens führen, wenn weniger Steuergrößen benötigt werden als Sensordaten empfangen werden. Eine weitere Verringerung lässt sich erreichen, wenn die Häufigkeit der Steuergrößenberechnung geringer gewählt wird als die Häufigkeit des Sensordatenempfangs.

Entsprechend ist erfindungsgemäß die Datenbrille konfiguriert, die Steuergrößen zu empfangen und die virtuelle 3D-Szene (Virtual Reality, VR) abhängig von den empfangenen Steuergrößen zu berechnen. Eine virtuelle 3D-Szene benötigt zu ihrer Darstellung üblicherweise ein großes Datenvolumen. Wenn eine virtuelle 3D-Szene einer Kinetose entgegenwirken soll, muss dieses Datenvolumen in Echtzeit konsistent zu den Beschleunigungen des Fahrzeugs berechnet und in der Datenbrille zur Anzeige gebracht werden. Die Erfindung beruht auf der Überlegung, die Datenbrille zu konfigurieren, eine virtuelle 3D-Szene abhängig von Steuergrößen zu berechnen. Auf diese Weise müssen keine datenintensiven virtuellen 3D-Szenen an die Datenbrille übertragen werden, um diese in der Datenbrille darzustellen. Vielmehr ist das Übertragen von wenigen für die Berechnung relevanten Steuergrößen ausreichend, wozu eine deutlich geringere Bandbreite benötigt wird.

In einer Ausführungsform weisen das Fahrzeug und die Datenbrille jeweils zueinander komplementäre drahtlose Kommunikationsschnittstellen auf, insbesondere IR-Schnittstellen, Bluetooth-Schnittstellen, WiFi-Schnittstellen und/oder NFC-Schnittstellen. Von den genannten Kommunikationsschnittstellen abweichende bekannte oder zukünftige Kommunikationsschnittstellen liegen innerhalb des Schutzbereichs der Erfindung. Selbstverständlich können das Fahrzeug und die Datenbrille auch mehrere komplementäre Schnittstellenpaare aufweisen, aus denen der Träger der Datenbrille ein beliebiges Schnittstellenpaar auswählen kann, um die Datenbrille mit dem Fahrzeug zu verbinden.

In einer bevorzugten Ausführungsform umfassen die von der Steuerungseinheit berechneten Steuergrößen eine absolute Position, einen Winkel zum Horizont, eine Querbeschleunigung und eine Längsbeschleunigung des Fahrzeugs und/oder ist die Datenbrille konfiguriert, die empfangenen Steuergrößen vor dem Berechnen einer virtuellen 3D-Szene nachzubearbeiten, insbesondere zu glätten. Mit anderen Worten sind vier Steuergrößen ausreichend, um virtuelle 3D-Szenen konsistent zu den Beschleunigungen des Fahrzeugs zu berechnen. Die absolute Position des Fahrzeugs kann zum räumlichen Anordnen innerhalb der virtuellen 3D-Szene verwendet werden. Anhand des Winkels zum Horizont lässt sich die Perspektive des Trägers der Datenbrille auf die virtuelle 3D-Szene korrespondierend zu einer realen Blickrichtung simulieren. Die Längs- und Querbeschleunigungen stellen die wichtigsten Steuergrößen für eine Verringerung der Kinetosegefahr dar. Sie können verwendet werden, um die virtuelle 3D-Szene konsistent mit dem Fahrzeug zu beschleunigen, wodurch sich eine Diskrepanz zwischen der visuellen Wahrnehmung und der vestibulären Wahrnehmung verringern oder vermeiden lässt.

In einer weiteren Ausführungsform umfasst das System ein mit dem Fahrzeug und/oder der Datenbrille drahtlos verbindbares mobiles Endgerät zum Anzeigen von Fahrzeugparametern und/oder Fahrtparametern, insbesondere eine Smartwatch, und ist die Datenbrille konfiguriert, ein zu dem mobilen Endgerät korrespondierendes virtuelles Endgerät in eine berechnete virtuelle 3D-Szene zu integrieren, so dass die von dem mobilen Endgerät angezeigten Fahrzeugparameter und/oder Fahrtparameter auf dem virtuellen Endgerät sichtbar sind. Das gleichzeitige Nutzen eines mobilen Endgeräts und seiner virtuellen Entsprechung schafft für den Träger der Datenbrille ein die Grenze zwischen der realen Welt und der virtuellen Welt überwindendes einheitliches Interaktionserleben. Eine Smartwatch eignet sich dazu in besonderer Weise, weil der Träger diese kontinuierlich an einem Handgelenk trägt und ihre Nutzung sehr komfortabel ist.

In einer Ausführungsform ist die Datenbrille konfiguriert, einen visuellen Hinweis, insbesondere einen metaphorischen Hinweis, an einen Träger der Datenbrille, insbesondere einen Fahrer des Fahrzeugs, in eine berechnete virtuelle 3D-Szene zu integrieren, um den Träger der Datenbrille zum Abnehmen der Datenbrille aufzufordern. Beispielsweise kann die berechnete virtuelle 3D-Szene ein Portal umfassen, welches virtuell passiert wird, wenn der Träger die Datenbrille abnehmen soll. Auf diese Weise wird für den Träger der Datenbrille ein sehr natürliches Interaktionserleben ermöglicht.

In einer weiteren Ausführungsform umfasst die Datenbrille mindestens einen optischen Sensor, insbesondere eine Kamera, zum Erfassen einer Umgebung der Datenbrille und ein digitales Modell einer Innenumgebung des Fahrzeugs, und ist die Datenbrille konfiguriert, mittels des digitalen Modells zwischen statischen Anteilen der erfassten Umgebung, welche zu der Innenumgebung korrespondieren, und dynamischen Anteilen der erfassten Umgebung, welche zu einer durch ein Fahrzeugfenster sichtbaren bewegten Außenumgebung des Fahrzeugs korrespondieren, zu unterscheiden. Mittels der erfassten dynamischen Anteile lässt sich die Bewegung der Datenbrille relativ zu einer Umgebung des Fahrzeugs bestimmen. Das zusätzliche Berücksichtigen der Relativbewegung zwischen der Datenbrille und der Umgebung kann die Diskrepanz zwischen visueller Wahrnehmung und vestibulärer Wahrnehmung weiter verringern.

In einer Ausführungsform ist das Fahrzeug konfiguriert, Verstellbereiche von Fahrzeugkomponenten, insbesondere einen positionellen Verstellbereich eines Vordersitzes, abhängig von dem Verwenden der Datenbrille unterschiedlich vorzusehen. Bei dem Nutzen einer Datenbrille ist beispielsweise der Abstand zwischen der an dem Kopf des Trägers angebrachten Datenbrille und einem Airbag des Fahrzeugs gegenüber dem Abstand zwischen dem Kopf des Trägers und dem Airbag des Fahrzeugs infolge des Raumbedarfs der Datenbrille verringert, infolgedessen der ausgelöste Airbag einen Impuls auf die Datenbrille und mittelbar den Kopf des Trägers übertragen kann, was mit einer Verletzungsgefahr für den Insassen einhergehen kann. Dies kann dadurch verhindert werden, dass der Träger einer Datenbrille seinen Fahrzeugsitz weniger weit nach vorn verstellen kann als ein Insasse des Fahrzeugs, der keine Datenbrille trägt. Mit anderen Worten kann der Verstellbereich eines Fahrzeugsitzes in seinem vorderen Bereich reduziert sein, wenn der auf dem Fahrzeugsitz sitzende Insasse eine Datenbrille trägt.

In einer weiteren Ausführungsform ist die Datenbrille konfiguriert, Informationen, welche Objekte in einer Umgebung des Fahrzeugs betreffen, in eine berechnete virtuelle 3D-Szene zu integrieren, insbesondere metaphorisch. Beispielsweise können Sehenswürdigkeiten, d.h. interessante Punkte (Point Of Interest, POI) oder interessante Produkte (Product Placement) von Anbietern in der Umgebung des Fahrzeugs in die virtuelle 3D-Szene integriert werden. Dabei kann eine zum Thema der virtuellen 3D-Szene passende Metaphorik verwendet werden.

In einer bevorzugten Ausführungsform ist die Datenbrille konfiguriert, einen zweidimensionalen Bewegtbildstrom, insbesondere einen Spielfilm, auf einem virtuellen Bildschirm abzuspielen und den virtuellen Bildschirm in einer berechneten virtuellen 3D-Szene darzustellen, welche inhaltlich zu den zweidimensionalen Bewegtbildern korrespondiert. Mit anderen Worten kann für die virtuelle 3D-Szene ein zu dem auf dem virtuellen Bildschirm abgespielten Bewegtbildstrom passendes Thema ausgewählt werden. Dies ermöglicht dem Träger der Datenbrille ein besonders homogenes Erleben.

Ein weiterer Gegenstand der Erfindung ist eine Datenbrille für ein Fahrzeug, welche ausgebildet und konfiguriert ist, mit dem Fahrzeug ein erfindungsgemäßes System zu bilden. Eine solche Datenbrille lässt sich drahtlos mit der Steuerungseinheit des Fahrzeugs verbinden, um einem die Datenbrille tragenden Insassen des Fahrzeugs ein kinetosefreies Betrachten eines in eine virtuelle 3D-Szene eingebetteten digitalen Inhalts zu ermöglichen und dabei mit einer geringen Bandbreite der drahtlosen Verbindung auszukommen.

Gegenstand der vorliegenden Erfindung ist auch ein Verfahren zum Darstellen eines digitalen Inhalts in einem Fahrzeug, insbesondere mittels eines erfindungsgemäßen Systems, bei dem von einer Steuerungseinheit, welche in dem Fahrzeug vorgesehen ist, aus Sensordaten, welche von mehreren in dem Fahrzeug vorgesehenen Sensoren empfangen werden, Steuergrößen berechnet werden, die berechneten Steuergrößen von dem Fahrzeug an eine mit dem Fahrzeug drahtlos verbundene Datenbrille übertragen werden und von der Datenbrille eine den digitalen Inhalt integrierende virtuelle 3D-Szene abhängig von den empfangenen Steuergrößen berechnet wird. Bei dem erfindungsgemäßen Verfahren erfolgt die Berechnung der virtuellen 3D-Szene nicht fahrzeugseitig, sondern durch die Datenbrille. Infolgedessen muss kein großes Datenvolumen von dem Fahrzeug an die Datenbrille übertragen werden. Für eine mit Beschleunigungen des Fahrzeugs konsistente virtuelle 3D-Szene sind vielmehr wenige aus den Sensordaten berechnete Steuergrößen ausreichend, deren kontinuierliche Übertragung entsprechend eine geringe Bandbreite für die drahtlose Verbindung zwischen dem Fahrzeug und der Datenbrille benötigt.

Die Erfindung ist nachfolgend anhand einer Ausführungsform des erfindungsgemäßen Systems zum kinetosefreien Betrachten eines digitalen Inhalts durch einen Insassen in einem Fahrzeug dargestellt und weiter beschrieben.

Das System umfasst ein Fahrzeug mit einer in dem Fahrzeug vorgesehenen Steuerungseinheit und mehreren in dem Fahrzeug vorgesehenen Sensoren, die mit der Steuerungseinheit verbunden sind. Ferner umfasst das System eine Datenbrille zum Darstellen einer den digitalen Inhalt integrierenden virtuellen 3D-Szene. Das Fahrzeug, insbesondere die in dem Fahrzeug vorgesehenen Sensoren und die Datenbrille weisen zueinander komplementäre drahtlose WiFi-Kommunikationsschnittstellen auf, mittels derer sie miteinander verbunden sind.

Die Steuerungseinheit ist konfiguriert, aus von den Sensoren empfangenen Sensordaten im Rahmen einer Vorverarbeitung Steuergrößen für die Datenbrille zu berechnen und die Steuergrößen an die Datenbrille mittels der WiFi-Kommunikationsschnittstelle drahtlos zu übertragen. Die Steuergrößen umfassen eine absolute Position, einen Winkel zum Horizont, eine Querbeschleunigung und eine Längsbeschleunigung des Fahrzeugs.

Die Datenbrille ist konfiguriert ist, die von der Steuerungseinheit berechneten Steuergrößen zu empfangen, nachzubearbeiten und abhängig von den empfangenen und nachbearbeiteten Steuergrößen die virtuelle 3D-Szene zu berechnen. Die Nachbearbeitung der zu diskreten Zeitpunkten empfangenen Steuergrößen dient dazu, durch Glätten einen kontinuierlichen Verlauf der Steuergrößen zu interpolieren.

Weiterhin umfasst das System eine Smartwatch zum Anzeigen von Fahrzeug- oder Fahrtparametern, wie beispielsweise einer verbleibenden Entfernung zu einem Fahrtziel oder einem verfügbaren Energievorrat. Die Smartwatch weist eine drahtlose WiF-Kommunikationsschnittstelle auf und ist mit dem Fahrzeug oder der Datenbrille drahtlos verbunden. Die Datenbrille ist konfiguriert, eine zu der Smartwatch korrespondierende virtuelle Smartwatch in die berechnete virtuelle 3D-Szene zu integrieren, so dass die von der Smartwatch angezeigten Fahrzeugparameter bzw. Fahrtparameter auch auf der virtuellen Smartwatch sichtbar sind.

Die Datenbrille ist konfiguriert, einen visuellen Hinweis, insbesondere einen metaphorischen Hinweis, für einen Träger der Datenbrille, insbesondere für einen Fahrer des Fahrzeugs, in die berechnete virtuelle 3D-Szene zu integrieren, um den Träger der Datenbrille zum Abnehmen der Datenbrille aufzufordern. Als metaphorischen Hinweis stellt die Datenbrille ein Portal dar, welches von dem die Datenbrille tragenden Fahrer des Fahrzeugs virtuell passiert wird.

Die Datenbrille umfasst eine Kamera zum Erfassen einer Umgebung der Datenbrille und ein digitales Modell einer Innenumgebung des Fahrzeugs.

Sie ist konfiguriert, mittels des digitalen Modells zwischen statischen Anteilen der erfassten Umgebung, welche zu der Innenumgebung, d.h. zu dem Inneren des Fahrzeugs, korrespondieren, und dynamischen Anteilen der erfassten Umgebung, welche zu einer durch ein Fahrzeugfenster sichtbaren relativ bewegten Außenumgebung des Fahrzeugs korrespondieren, zu unterscheiden.

Das Fahrzeug ist konfiguriert, einen positionellen Verstellbereich eines Vordersitzes abhängig davon unterschiedlich vorzusehen, ob der auf dem Vordersitz sitzende Insasse eine Datenbrille trägt oder nicht.

Die Datenbrille ist ferner konfiguriert, Informationen, welche Objekte in einer Umgebung des Fahrzeugs betreffen, in die berechnete virtuelle 3D-Szene zu integrieren, insbesondere metaphorisch. Dazu werden beispielsweise Standorte bestimmter Anbieter in der Umgebung des Fahrzeugs, spezielle Werbehinweise oder touristische Sachinformationen zu Sehenswürdigkeiten in der Umgebung des Fahrzeugs passend zu dem Thema der virtuellen 3D-Szene dargestellt.

Die Datenbrille ist konfiguriert, einen Spielfilm auf einem in die virtuelle 3D-Szene integrierten virtuellen Bildschirm abzuspielen. Dabei wird der virtuelle Bildschirm in einer berechneten virtuellen 3D-Szene dargestellt, welche thematisch zu den zweidimensionalen Bewegtbildern korrespondiert. Beispielsweise kann die virtuelle 3D-Szene einen Flug über den aus den Star Wars-Spielfilmen bekannten Todesstern simulieren, während auf dem virtuellen Bildschirm eine Episode von einem der Star Wars-Spielfilme abgespielt wird.

Während des Betriebs des erfindungsgemäßen Systems werden von der Steuerungseinheit aus Sensordaten Steuergrößen berechnet und an die mit dem Fahrzeug drahtlos verbundene Datenbrille übertragen. Die Datenbrille berechnet abhängig von den empfangenen Steuergrößen eine den digitalen Inhalt integrierende virtuelle 3D-Szene.

Ein Vorteil des erfindungsgemäßen Systems besteht darin, dass für einen eine Datenbrille tragenden Insassen eines Fahrzeugs ein kinetosefreies Betrachten eines digitalen Inhalts im Rahmen einer auf den digitalen Inhalt thematisch abgestimmten datenintensiven VR-Anwendung ermöglicht wird, ohne für eine drahtlose Verbindung zwischen der Datenbrille und dem Fahrzeug eine große Bandbreite bereitzuhalten. Dadurch wird dem Träger der Datenbrille ein homogenes und ganzheitliches Erleben des digitalen Inhalts bei gleichzeitig einfacher Handhabbarkeit geboten. Dieser Vorteil vergrößert sich noch, wenn mehrere Insassen eines Fahrzeugs gleichzeitig unterschiedliche digitale Inhalte betrachten.

## Patentansprüche

1. System zum kinetosefreien Betrachten eines digitalen Inhalts in einem Fahrzeug, welches ein Fahrzeug mit einer Steuerungseinheit und mehreren mit der Steuerungseinheit verbundenen Sensoren und eine mit der Steuerungseinheit drahtlos verbindbare Datenbrille zum Darstellen einer den digitalen Inhalt integrierenden virtuellen 3D-Szene umfasst, bei dem die Steuerungseinheit konfiguriert ist, aus von den Sensoren empfangenen Sensordaten Steuergrößen zu berechnen und die Steuergrößen an die Datenbrille zu übertragen, und bei dem die Datenbrille konfiguriert ist, die Steuergrößen zu empfangen und die virtuelle 3D-Szene abhängig von den empfangenen Steuergrößen zu berechnen.

2. System nach Anspruch 1, bei dem das Fahrzeug und die Datenbrille jeweils zueinander komplementäre drahtlose Kommunikationsschnittstellen aufweisen, insbesondere IR-Schnittstellen, Bluetooth-Schnittstellen, WiFi-Schnittstellen und/oder NFC-Schnittstellen.

3. System nach einem der Ansprüche 1 oder 2, bei dem die von der Steuerungseinheit berechneten Steuergrößen eine absolute Position, einen Winkel zum Horizont, eine Querbeschleunigung und eine Längsbeschleunigung des Fahrzeugs umfassen und/oder bei dem die Datenbrille konfiguriert ist, die empfangenen Steuergrößen vor dem Berechnen einer virtuellen 3D-Szene nachzubearbeiten, insbesondere zu glätten.

4. System nach einem der Ansprüche 1 oder 2, welches ein mit dem Fahrzeug und/oder der Datenbrille drahtlos verbindbares mobiles Endgerät zum Anzeigen von Fahrzeugparametern und/oder Fahrtparametern, insbesondere eine Smartwatch, umfasst und bei dem die Datenbrille konfiguriert ist, ein zu dem mobilen Endgerät korrespondierendes virtuelles Endgerät in die berechnete virtuelle 3D-Szene zu integrieren, so dass die von dem mobilen Endgerät angezeigten Fahrzeugparameter und/oder Fahrtparameter auf dem virtuellen Endgerät sichtbar sind.

5. System nach einem der Ansprüche 1 oder 2, bei dem die Datenbrille konfiguriert ist, einen visuellen Hinweis, insbesondere einen metaphorischen Hinweis, für einen Träger der Datenbrille, insbesondere einen Fahrer des Fahrzeugs, in die berechnete virtuelle 3D-Szene zu integrieren, um den Träger der Datenbrille zum Abnehmen der Datenbrille aufzufordern.

6. System nach einem der Ansprüche 1 oder 2, bei dem die Datenbrille mindestens einen optischen Sensor, insbesondere eine Kamera, zum Erfassen einer Umgebung der Datenbrille und ein digitales Modell einer Innenumgebung des Fahrzeugs umfasst, bei dem die Datenbrille konfiguriert ist, mittels des digitalen Modells zwischen statischen Anteilen der erfassten Umgebung, welche zu der Innenumgebung korrespondieren, und dynamischen Anteilen der erfassten Umgebung, welche zu einer durch ein Fahrzeugfenster sichtbaren bewegten Außenumgebung des Fahrzeugs korrespondieren, zu unterscheiden.

7. System nach einem der Ansprüche 1 oder 2, bei dem das Fahrzeug konfiguriert ist, Verstellbereiche von Fahrzeugkomponenten, insbesondere einen positionellen Verstellbereich eines Vordersitzes, abhängig von dem Verwenden der Datenbrille unterschiedlich vorzusehen.

8. System nach einem der Ansprüche 1 oder 2, bei dem die Datenbrille konfiguriert ist, Informationen, welche Objekte in einer Umgebung des Fahrzeugs betreffen, in die berechnete virtuelle 3D-Szene zu integrieren, insbesondere metaphorisch, und/oder einen zweidimensionalen Bewegtbildstrom, insbesondere einen Spielfilm, auf einem virtuellen Bildschirm abzuspielen und den virtuellen Bildschirm in einer berechneten virtuellen 3D-Szene darzustellen, welche inhaltlich zu den zweidimensionalen Bewegtbildern korrespondiert.

9. Datenbrille für ein Fahrzeug, welche ausgebildet und konfiguriert ist, mit dem Fahrzeug ein System nach einem der Ansprüche 1 bis 8 zu bilden.

10. Verfahren zum Darstellen eines digitalen Inhalts in einem Fahrzeug, insbesondere mittels eines Systems nach einem der Ansprüche 1 bis 8, bei dem von einer Steuerungseinheit, welche in dem Fahrzeug vorgesehen ist, aus Sensordaten, welche von mehreren in dem Fahrzeug vorgesehenen Sensoren empfangen werden, Steuergrößen berechnet werden, die berechneten Steuergrößen von dem Fahrzeug an eine mit dem Fahrzeug drahtlos verbundene Datenbrille übertragen werden und von der Datenbrille eine den digitalen Inhalt integrierende virtuelle 3D-Szene abhängig von den empfangenen Steuergrößen berechnet wird.

## Claims

1. A system for motion sickness-free observation of a digital content in a vehicle, comprising a vehicle with a control unit and multiple sensors connected to the control unit, and smartglasses that can be wirelessly connected to the control unit for displaying a virtual 3D scene that integrates the digital content, wherein the control unit is configured for calculating control parameters from sensor data received from the sensors and for transferring the control parameters to the smartglasses, and wherein the smartglasses are configured for receiving the control parameters and calculating the virtual 3D scene dependent on the received control parameters.

2. The system according to claim 1, wherein the vehicle and the smartglasses respectively have wireless communication interfaces complementary to one another, in particular IR interfaces, Bluetooth interfaces, WiFi interfaces and/or NFC interfaces.

3. The system according to any one of claims 1 or 2, wherein the control parameters calculated by the control unit comprise an absolute position, an angle to the horizon, a transverse acceleration and a longitudinal acceleration of the vehicle, and/or wherein the smartglasses are configured for post-processing, in particular for smoothing, the received control parameters before calculation of a virtual 3D scene.

4. The system according to any one of claims 1 or 2, comprising a mobile terminal for displaying vehicle parameters and/or trip parameters that can be wirelessly connected to the vehicle and/or the smartglasses, in particular a smart watch, and wherein the smartglasses are configured for integrating a virtual terminal corresponding to the mobile terminal into the calculated virtual 3D scene, such that the vehicle parameters and/or trip parameters displayed by the mobile terminal are visible on the virtual terminal.

5. The system according to any one of claims 1 or 2, wherein the smartglasses are configured for integrating a visual notification, in particular a metaphorical notification, for a wearer of the smartglasses, in particular a driver of the vehicle, into the calculated virtual 3D scene for requesting the wearer of the smartglasses to remove the smartglasses.

6. The system according to any one of claims 1 or 2, wherein the smartglasses comprise at least one optical sensor, in particular a camera, for detecting an environment of the smartglasses, and a digital model of an interior environment of the vehicle, wherein the smartglasses are configured for distinguishing, by means of the digital model, between static components of the detected environment corresponding to the interior environment and dynamic components of the detected environment corresponding to a moving exterior environment of the vehicle visible through a vehicle window.

7. The system according to any one of claims 1 or 2, wherein the vehicle is configured for providing adjustment ranges of vehicle components, in particular a position adjustment range of a front seat, differently dependent on the use of the smartglasses.

8. The system according to any one of claims 1 or 2, wherein the smartglasses are configured for integrating information relating to objects in an environment of the vehicle into the calculated virtual 3D scene, in particular metaphorically, and/or for playing a two-dimensional video stream, in particular a movie, on a virtual screen and for depicting the virtual screen in a calculated virtual 3D scene that corresponds in terms of content to the two-dimensional video.

9. Smartglasses for a vehicle that are designed and configured for forming a system according to one of claims 1 to 8 with the vehicle.

10. A method for displaying a digital content in a vehicle, in particular by means of a system according to one of claims 1 to 8, wherein control parameters are calculated by a control unit provided in the vehicle based on sensor data received from multiple sensors provided in the vehicle, the calculated control parameters are transferred from the vehicle to smartglasses wirelessly connected to the vehicle, and a virtual 3D scene integrating the digital content is calculated by the smartglasses dependent on the received control parameters.

## Revendications

1. Système d'observation sans cinétose d'un contenu numérique dans un véhicule, comprenant un véhicule doté d'une unité de commande et de plusieurs capteurs reliés à l'unité de commande et des lunettes de données pouvant être reliées sans fil à l'unité de commande pour représenter une scène virtuelle 3D intégrant le contenu numérique, l'unité de commande étant configurée pour calculer des grandeurs de commande à partir de données de capteurs reçues des capteurs et pour transmettre les grandeurs de commande aux lunettes de données, les lunettes de données étant configurées pour recevoir les grandeurs de commande et pour calculer la scène virtuelle 3D en fonction des grandeurs de commande reçues.

2. Système selon la revendication 1, le véhicule et les lunettes de données présentant des interfaces de communication sans fil respectivement complémentaires entre elles, en particulier des interfaces IR, des interfaces Bluetooth, des interfaces WiFi et/ou des interfaces NFC.

3. Système selon l'une des revendications 1 ou 2, les grandeurs de commande calculées par l'unité de commande comprenant une position absolue, un angle par rapport à l'horizon, une accélération transversale et une accélération longitudinale du véhicule et/ou les lunettes de données étant configurées pour retoucher, notamment pour lisser, les grandeurs de commande reçues avant le calcul d'une scène virtuelle 3D.

4. Système selon l'une des revendications 1 ou 2, comprenant un terminal mobile pouvant être connecté sans fil au véhicule et/ou aux lunettes de données pour afficher des paramètres du véhicule et/ou des paramètres de conduite, notamment une montre intelligente, et les lunettes de données étant configurées pour intégrer un terminal virtuel correspondant au terminal mobile dans la scène virtuelle 3D calculée, afin que les paramètres du véhicule et/ou les paramètres de conduite affichés par le terminal mobile soient visibles sur le terminal virtuel.

5. Système selon l'une des revendications 1 ou 2, les lunettes de données étant configurées pour intégrer une indication visuelle, notamment une indication métaphorique, destinée à un porteur des lunettes de données, notamment un conducteur du véhicule, dans la scène virtuelle 3D calculée, afin d'inviter le porteur des lunettes de données à retirer les lunettes de données.

6. Système selon l'une des revendications 1 ou 2, les lunettes de données comprenant au moins un capteur optique, notamment une caméra, pour détecter un environnement des lunettes de données et un modèle numérique d'un environnement intérieur du véhicule, les lunettes de données étant configurées pour faire la distinction, au moyen du modèle numérique, entre des parties statiques de l'environnement détecté, qui correspondent à l'environnement intérieur, et des parties dynamiques de l'environnement détecté, qui correspondent à un environnement extérieur du véhicule en mouvement, visible à travers une fenêtre du véhicule.

7. Système selon l'une des revendications 1 ou 2, le véhicule étant configuré pour prévoir des plages de réglage de composants du véhicule, notamment une plage de réglage de position d'un siège avant, différemment en fonction de l'utilisation des lunettes de données.

8. Système selon l'une des revendications 1 ou 2, les lunettes de données étant configurées pour intégrer des informations concernant des objets dans un environnement du véhicule dans la scène virtuelle 3D calculée, en particulier métaphoriquement, et/ou pour lire un flux d'images animées bidimensionnelles, notamment un film, sur un écran virtuel et pour afficher l'écran virtuel dans une scène virtuelle 3D calculée dont le contenu correspond à celui des images animées bidimensionnelles.

9. Lunettes de données pour un véhicule, qui sont conçues et configurées pour former avec le véhicule un système selon l'une des revendications 1 à 8.

10. Procédé de représentation d'un contenu numérique dans un véhicule, notamment au moyen d'un système selon l'une des revendications 1 à 8, dans lequel des grandeurs de commande sont calculées par une unité de commande prévue dans le véhicule à partir de données de capteurs reçues de plusieurs capteurs prévus dans le véhicule, les grandeurs de commande calculées sont transmises par le véhicule à des lunettes de données reliées sans fil au véhicule et une scène virtuelle 3D intégrant le contenu numérique est calculée par les lunettes de données en fonction des grandeurs de commande reçues.
